# EUROPEAN PATENT APPLICATION

(11) **EP 3 040 037 A1**
(43) Date of publication of application: **06.07.2016**
(21) Application number: 14840897.4
(22) Date of filing: 21.08.2014
(51) Int. Cl.: A61B 17/56, A61B 17/14, A61F 2/32, A61F 2/46

(54) **BONE-CUTTING GUIDE POSITIONING DEVICE**

(30) Priority: 29.08.2013 JP 2013178123
(71) Applicant: Coco Inc., Kanazawa-shi, Ishikawa 921-8034 (JP); Maeda, Toru, Kanazawa-shi, Ishikawa 920-0849 (JP); Kanekasu, Koichi, Takaoka-shi Toyama 933-0874 (JP)
(72) Inventor: MAEDA, Toru, Kanazawa-shi Ishikawa 920-0849 (JP); KANEKASU, Koichi, Takaoka-shi Toyama 933-0874 (JP)
(74) Representative: Vossius & Partner Patentanwälte Rechtsanwälte mbB
(86) International application number: PCT/JP2014/004308
(87) International publication number: WO 2015/029396

(57) **Abstract**

A bone resection guide positioning apparatus (10) includes: a fixing part (14) including a medullary rod (22) configured to be inserted into a femur, a base (24) provided at one end of the medullary rod (22) and configured to be disposed in proximity to a distal end of the femur, and a supporting pillar (26) detachably connected to the base (24); a measuring part (16) including a first connection portion (52) connected to the supporting pillar (26) and an indicator (50) coupled to the first connection portion (52), the indicator (50) being configured to indicate a valgus angle formed by a femoral functional axis and a femoral anatomical axis based on a positional relationship between the indicator (50) and a femoral head center, the femoral functional axis connecting the femoral head center and a knee joint center; and a guide support (18) including a second connection portion (80) connected to the base (24) and a support body (82) connected to the second connection portion (80), to which support body (82) a bone resection guide is detachably mounted.

## Description

### Technical Field

The present invention relates to a bone resection guide positioning apparatus for positioning a bone resection guide at a proper position on a femur, the bone resection guide guiding a bone saw.

### Background Art

Artificial knee joint replacement, in which a knee joint is replaced with an artificial joint, is an excellent surgical treatment for reducing aching pain and limitation on the daily life activity due to advanced knee osteoarthritis. However, since an artificial knee joint implant used in the artificial knee joint replacement is an artificial object, the artificial knee joint implant has a certain limit in terms of its durability, and there are cases where the surgery becomes necessary again due to abrasion of a cushion portion and/or looseness of the implant. When viewing an X-ray image (a frontal plane image) of the anterior surface of a lower extremity, if the angle of intersection between a functional axis of a femur, the functional axis connecting the femoral head center to the knee joint center, and a functional axis of ossa cruris, the functional axis connecting the knee joint center to the talus center (this angle is hereinafter referred to as a "lower extremity alignment angle"), is zero degree, the weight bearing axis of the human body passes through the knee joint center. In this case, the balance when standing on the leg is desirable. As a result, not only is the life of the artificial knee joint extended, but the function of the lower extremity is improved, which makes it possible to improve the patient satisfaction. For this reason, in the artificial knee joint replacement, it is necessary to perform bone resection at the knee joint with high precision in order to bring the lower extremity alignment angle after the surgery as close as possible to zero degree (preferably, less than ± 3 degrees).

By use of a navigation system as disclosed in Patent Literature 1, the position of the femoral head center can be precisely specified by a computer even during the surgery. Therefore, a bone resection guide can be precisely positioned at a proper position on the femur while confirming the position of the femoral head center, and by use of the bone resection guide, the bone resection at the knee joint can be performed with high precision. However, the introduction cost and running cost of the navigation system are significantly high. In addition, a time for operating the navigation system is necessary during the surgery, and thereby the surgery time becomes long. For these problems, the use of such a navigation system is not widespread at present.

### Citation List

### Patent Literature

Japanese National Phase PCT Laid-Open Publication No. 2008-515601

### Summary of Invention

### Technical Problem

The present invention has been made in order to solve the above-described problems. An object of the present invention is to provide a bone resection guide positioning apparatus for performing bone resection in a simple and easy manner with high precision while reducing costs. for the surgery.

### Solution to Problem

In order to solve the above-described problems, a bone resection guide positioning apparatus according to the present invention includes: a fixing part including a medullary rod configured to be inserted into a femur, a base provided at one end of the medullary rod and configured to be disposed in proximity to a distal end of the femur, and a supporting pillar detachably connected to the base; a measuring part including a first connection portion connected to the supporting pillar and an indicator coupled to the first connection portion, the indicator being configured to indicate a valgus angle formed by a femoral functional axis and a femoral anatomical axis based on a positional relationship between the indicator and a femoral head center, the femoral functional axis connecting the femoral head center and a knee joint center; and a guide support including a second connection portion connected to the base and a support body connected to the second connection portion, to which support body the bone resection guide is detachably mounted, such that a mounting position of the bone resection guide mounted to the support body is adjustable.

When the medullary rod of the fixing part is inserted into the femur and the fixing part is fixed to the femur, the measuring part connected to the supporting pillar and the guide support connected to the base are positioned relative to the femur. Therefore, the valgus angle can be precisely measured by the measuring part connected to the supporting pillar. For the positioning of the bone resection guide on the femur, merely adjusting the position of the bone resection guide relative to the guide support connected to the base in accordance with the valgus angle will suffice. This makes it possible to readily and precisely position the bone resection guide at the proper position on the femur. For example, if the guide support includes a position adjusting mechanism that adjusts the mounting position of the bone resection guide mounted to the support body, the bone resection guide can be readily and precisely positioned at the proper position on the femur by operating the position adjusting mechanism in accordance with the valgus angle. It should be noted that the "proper position" herein means a suitable position of the bone resection guide for bringing the lower extremity alignment angle after the surgery close to zero degree. At the time of mounting the bone resection guide to the guide support, the supporting pillar of the fixing part can be removed from the base. Therefore, the supporting pillar will not be a hindrance.

### Advantageous Effects of Invention

According to the present invention, the bone resection guide can be precisely positioned at the proper position on the femur with a simple configuration that does not use a navigation system. This makes it possible to perform bone resection of the femur with high precision while reducing costs for artificial knee joint replacement.

### Brief Description of Drawings

Fig. 1 shows the structure of a femur and a tibia of a patient viewed in a direction perpendicular to a frontal plane.
Fig. 2 is a plan view showing a state where a bone resection guide positioning apparatus according to one embodiment is fixed to the femur.
Fig. 3 is a perspective view showing the configuration of the bone resection guide positioning apparatus according to the embodiment.
Fig. 4 is a plan view showing the configurations of a fixing part and a measuring part.
Fig. 5 is an exploded perspective view showing the configurations of the fixing part and a guide support (excluding a support body).
Fig. 6 is a perspective view showing a connection structure in which a base and a supporting pillar are connected.
Fig. 7 is an exploded perspective view showing the configuration of an indicator.
Fig. 8 is a perspective view showing the configuration of a connection portion (a first connection portion).
Fig. 9 is a side view showing a state where the fixing part and the measuring part are positioned relative to the femur.
Fig. 10 is a side view showing a state where a bone resection guide is mounted to the guide support.
Fig. 11 is a perspective view showing a step of resecting the femur.
Fig. 12 is a plan view showing a state where the measuring part is used on the right-side femur.
Fig. 13 is a perspective view showing a variation of a locking portion locked to the femur.
Fig. 14 is a perspective view showing another variation of the locking portion locked to the femur.

### Description of Embodiments

Hereinafter, artificial knee joint replacement is briefly described, and then a preferred embodiment of a bone resection guide positioning apparatus according to the present invention is described.

### (Brief Description of Artificial Knee Joint Replacement)

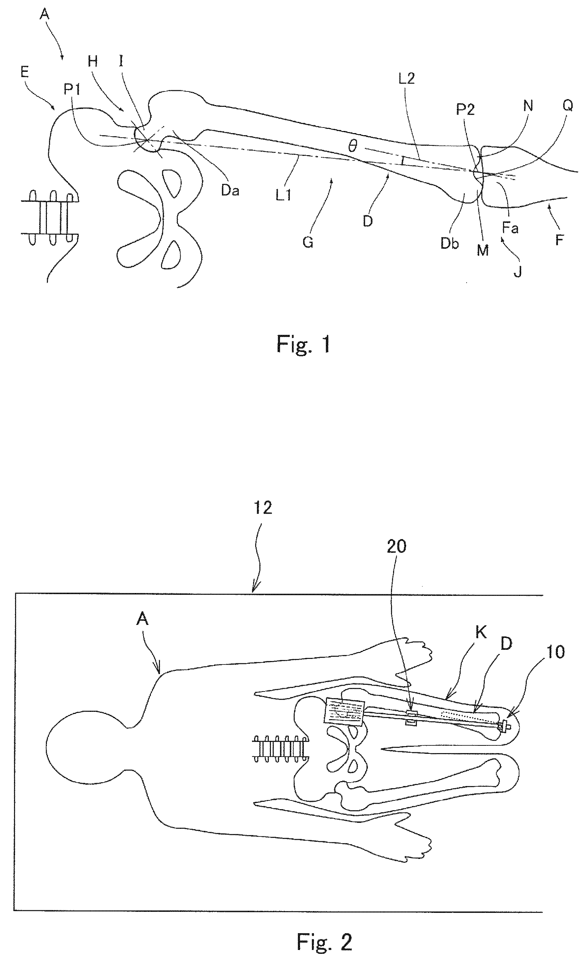

Fig. 1 shows the structure of a femur D and a tibia F of a patient A viewed in a direction perpendicular to a frontal plane. As shown in Fig. 1, the femur D is a bone that is positioned between the pelvis E and the tibia F and that forms part of a lower extremity G. A femoral head I forming part of a hip joint H is positioned at a femur proximal end Da. A medial condyle M and a lateral condyle N forming part of a knee joint J are positioned at a femur distal end Db. An intercondylar region Q positioned between the medial condyle M and the lateral condyle N has a recessed shape. The medial condyle M and the lateral condyle N are in contact with a tibia proximal end Fa with cartilage interposed therebetween. The artificial knee joint replacement is a surgery for cutting out the femur distal end Db that has deformed due to knee osteoarthritis, rheumatoid arthritis, or the like, and replacing the cut portion with an artificial joint.

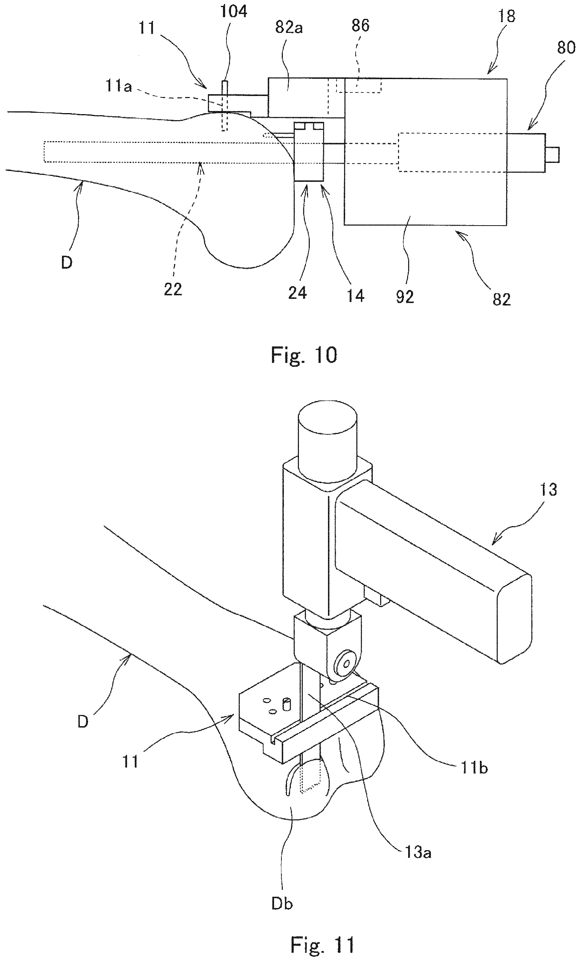

As shown in Fig. 1, a valgus angle θ is an angle of intersection between a femoral functional axis L1 and a femoral anatomical axis L2, the femoral functional axis L1 connecting a femoral head center P1 and a knee joint center P2. The valgus angle θ varies for each patient A. Therefore, in order to bring the lower extremity alignment angle after the surgery close to zero degree, it is necessary to insert a medullary rod along the femoral anatomical axis L2 and precisely cut out the femur distal end Db with a cross section corresponding to the valgus angle θ. By use of a bone resection guide positioning apparatus 10 according to the embodiment, a bone resection guide 11 (Fig. 11) can be precisely positioned at a proper position on the femur D in accordance with the valgus angle θ. As shown in Fig. 11, in a bone resection step, the femur distal end Db is cut out by a bone saw 13 in a manner to move a blade 13a of the bone saw 13 along a guide portion 11b of the bone resection guide 11.

### (Embodiment)

Fig. 2 is a plan view showing a state where the bone resection guide positioning apparatus 10 according to the embodiment is fixed to the femur D of the patient A. As shown in Fig. 2, in the artificial knee joint replacement, the patient A is laid on his or her back on a surgical table 12. Then, the bone resection guide positioning apparatus 10 is fixed to the femur D of the patient A. In the present embodiment, the surgical table 12 is placed such that the top surface thereof is parallel to a horizontal plane. The frontal plane of the patient A laid on the surgical table 12 is made parallel to the horizontal plane. That is, the direction perpendicular to the frontal plane coincides with the vertical direction.

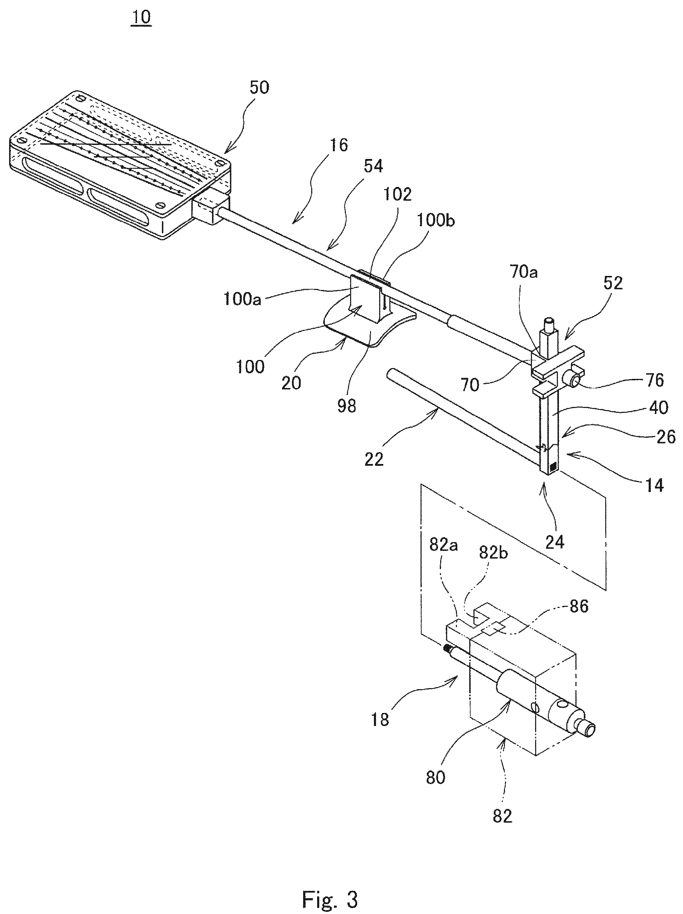

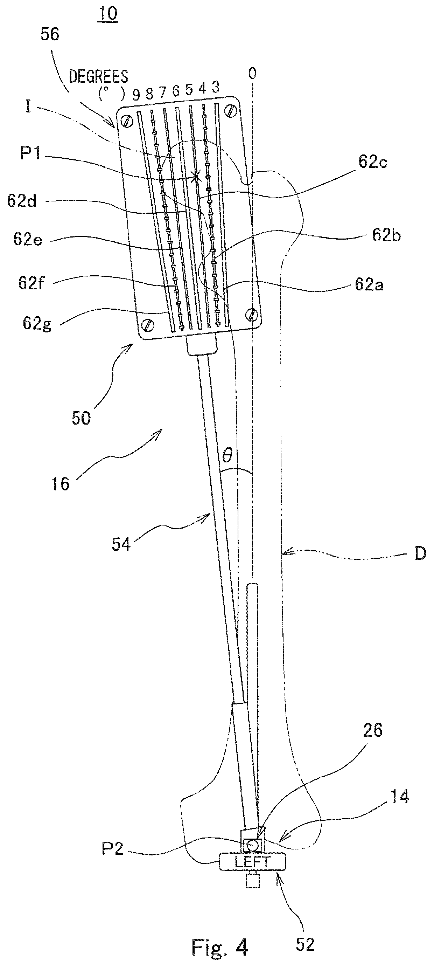

Fig. 3 is a perspective view showing the configuration of the bone resection guide positioning apparatus 10. Fig. 4 is a plan view showing the configurations of a fixing part 14 and a measuring part 16. As shown in Fig. 4, the bone resection guide positioning apparatus 10 includes: the fixing part 14 configured to be fixed to the femur D; and the measuring part 16 for measuring the valgus angle θ. In addition, as shown in Fig. 3, the bone resection guide positioning apparatus 10 includes: a guide support 18, which supports the bone resection guide 11 (Fig. 11); and a supporting member 20, which supports the measuring part 16.

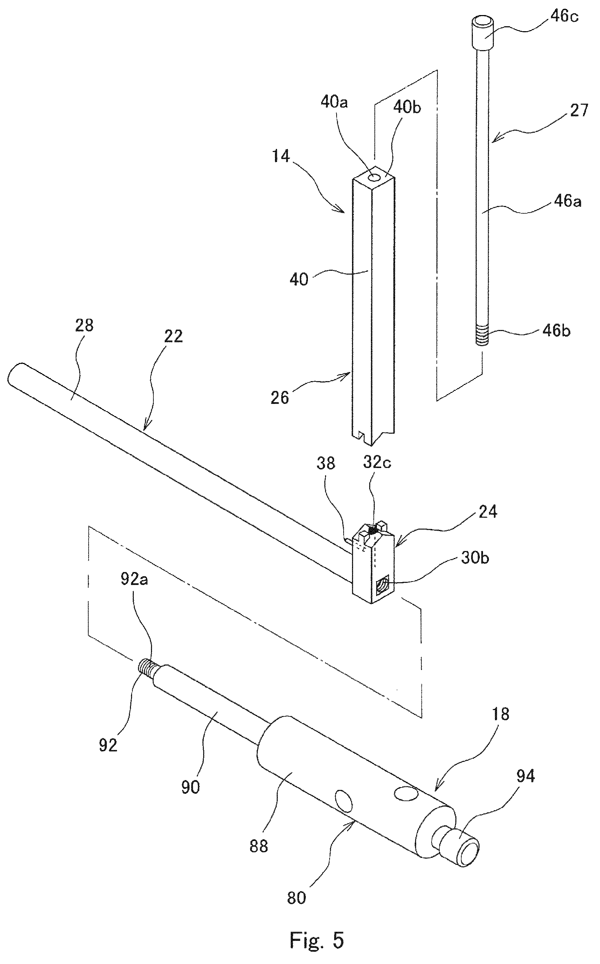

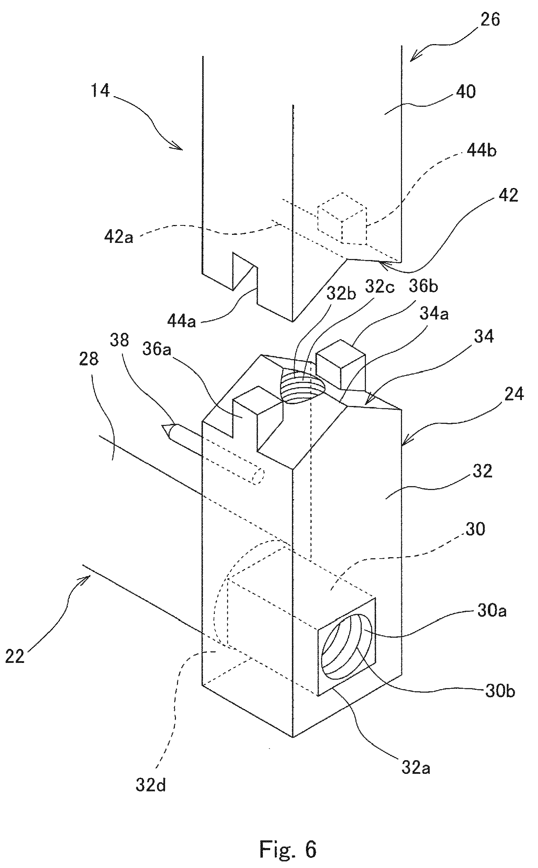

Fig. 5 is an exploded perspective view showing the configurations of the fixing part 14 and the guide support 18 (excluding a support body 82). Fig. 6 is a perspective view showing a connection structure in which a base 24 and a supporting pillar 26 are connected. As shown in Fig. 5, the fixing part 14 includes: a medullary rod 22 configured to be inserted into the femur D (Fig. 1) in parallel to the femoral anatomical axis L2 (Fig. 1); the base 24 provided at one end of the medullary rod 22 and configured to be disposed in proximity to the femur distal end Db (Fig. 1); the supporting pillar 26 detachably connected to the base 24; and a connecting member 27 connecting the base 24 and the supporting pillar 26 together.

As shown in Fig. 5, the medullary rod 22 includes a bar-like rod body 28 with a substantially round cross section. As shown in Fig. 6, a connection portion 30, which is connected to the base 24, is formed at one end of the rod body 28. The connection portion 30 has a non-round (in the present embodiment, quadrangular) cross section. In the connection portion 30, a hole 30a is formed in a manner extending in the axial direction of the rod body 28. A female screw 30b is formed on the inner peripheral surface of the hole 30a.

As shown in Fig. 6, the base 24 includes a base body 32, which is formed in a manner extending vertically and perpendicularly to the medullary rod 22. The base body 32 has a non-round (in the present embodiment, quadrangular) cross section. The connection portion 30 of the medullary rod 22 is fitted to the lower part of the base body 32, and a non-round (in the present embodiment, quadrangular) through-hole 32a, in which the connection portion 30 is fixed, is formed in a manner extending in a direction perpendicular to the axis of the base body 32. A protruding portion 34 protruding upward in an inverted V shape is formed on the upper face of the base body 32. An edge line 34a of the protruding portion 34 is formed to be parallel to the medullary rod 22. Substantially quadrangular fitting protrusions 36a and 36b protruding upward are formed at both sides of the protruding portion 34, respectively, with the edge line 34a disposed between the fitting protrusions 36a and 36b.

As shown in Fig. 6, a through-hole 32b is formed in the base body 32 in a manner extending in the axial direction (the vertical direction), and a female screw 32c serving as a "second screw" is formed on the inner peripheral surface of the through-hole 32b. A pin 38 serving as a "locking portion" and configured to be locked to the femur D (Fig. 1) is provided on an outer side surface 32d of the base body 32, on which the medullary rod 22 is provided. The pin 38 is formed to be parallel to the medullary rod 22, and the distal end of the pin 38 is sharp such that the pin 38 sticks into the femur D (Fig. 1).

As shown in Fig. 5, the supporting pillar 26 includes a supporting pillar body 40, which is formed in a manner extending vertically and perpendicularly to the medullary rod 22. The supporting pillar body 40 has a non-round (in the present embodiment, quadrangular) cross section. As shown in Fig. 6, a recessed portion 42, to which the protruding portion 34 of the base 24 is fitted, is formed in the lower face of the supporting pillar body 40, such that the recessed portion 42 is recessed upward in an inverted V shape. Fitting recesses 44a and 44b fitted to the fitting protrusions 36a and 36b are formed at both sides of the recessed portion 42, respectively, with a trough line 42a disposed between the fitting recesses 44a and 44b. As shown in Fig. 5, a through-hole 40a is formed in the supporting pillar body 40 in a manner extending in the axial direction (the vertical direction).

As shown in Fig. 5, the connecting member 27 includes: a shaft 46a formed to have a length greater than the length of the through-hole 40a; a male screw 46b formed at one end (lower end) of the shaft 46a and serving as a "first screw"; and a head 46c formed at the other end (upper end) of the shaft 46a. The head 46c receives rotational force imparted from, for example, hand fingers of a surgeon. At the time of connecting the supporting pillar 26 to the base 24, the connecting member 27 is inserted into the through-hole 40a from above, and the head 46c is locked to an upper end surface 40b of the supporting pillar body 40. Then, rotational force is imparted from the head 46c to the connecting member 27. As a result, the male screw 46b (the first screw) is screwed into the female screw 32c (the second screw). Consequently, the protruding portion 34 and the recessed portion 42 shown in Fig. 6 are fitted together, such that the fitting protrusions 36a and 36b and the fitting recesses 44a and 44b are fitted together, and thereby the supporting pillar 26 and the base 24 are positioned relative to each other and firmly connected to each other. It should be noted that, alternatively, the "first screw" may be a female screw, and the "second screw" may be a male screw, so long as the "first screw" and the "second screw" are configured to be screwed together.

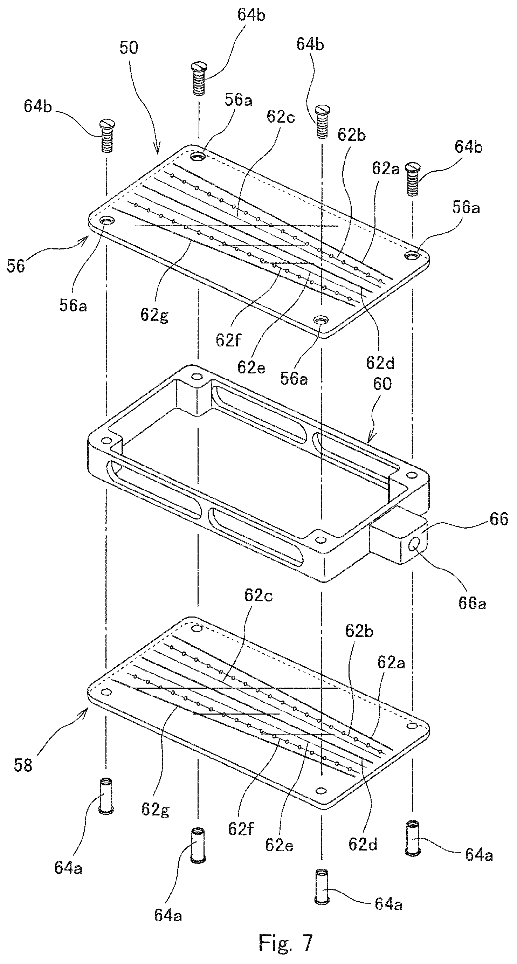

As shown in Fig. 4, the measuring part 16 includes: an indicator 50 configured to indicate the valgus angle θ based on a positional relationship between the indicator 50 and the femoral head center P1; a connection portion 52 connected to the fixing part 14 and serving as a "first connection portion"; and a rod-shaped coupling portion 54, which couples the indicator 50 and the connection portion 52 together. Fig. 7 is an exploded perspective view showing the configuration of the indicator 50. As shown in Fig. 7, the indicator 50 includes: a first plate-shaped portion 56 and a second plate-shaped portion 58, which are disposed such that they are spaced apart from each other in a direction perpendicular to the frontal plane (i.e., the vertical direction); and a spacer 60 disposed between the peripheral portion of the lower surface of the first plate-shaped portion 56 and the peripheral portion of the upper surface of the second plate-shaped portion 58. The indicator 50 further includes a plurality of gradations 62a to 62g provided in the first plate-shaped portion 56 and a plurality of gradations 62a to 62g provided in the second plate-shaped portion 58. The indicator 50 further includes: a plurality of (in the present embodiment, four) female screw members 64a; and a plurality of (in the present embodiment, four) male screw members 64b, which are screwed into the plurality of female screw members 64a, respectively.

As shown in Fig. 7, the first plate-shaped portion 56 is formed of a transparent material such as acrylic resin, and is a substantially quadrangular plate-shaped portion. A plurality of (in the present embodiment, four) through-holes 56a are formed in the peripheral portion of the first plate-shaped portion 56. The plurality of gradations 62a to 62g are provided in the first plate-shaped portion 56. It should be noted that the shape of the first plate-shaped portion 56 is not particularly limited, but may be a sector shape, triangular shape, trapezoidal shape, round shape, or so forth. The second plate-shaped portion 58 is formed in the same manner as the first plate-shaped portion 56.

As shown in Fig. 4, each of the plurality of gradations 62a to 62g provided in the first plate-shaped portion 56 is formed of, for example, a metal or synthetic resin and has a linear shape, and is embedded inside the first plate-shaped portion 56 such that a line extending from each of the plurality of gradations 62a to 62g passes through the center of the supporting pillar 26 (which coincides with the knee joint center P2). Among the plurality of gradations 62a to 62g, the central gradation 62d is disposed such that it is inclined relative to the medullary rod 22 toward the femoral head I by a predetermined angle (in the present embodiment, six degrees). A plurality of (in the present embodiment, three) gradations 62a to 62c are disposed at one side (outer side) relative to the central gradation 62d, and a plurality of (in the present embodiment, three) gradations 62e to 62g are disposed at the other side (inner side) relative to the central gradation 62d, such that the gradations 62a to 62c and the gradations 62e to 62g are disposed in a symmetrical manner with respect to the central gradation 62d.

As shown in Fig. 4, among the plurality of gradations 62a to 62g, a regular angular interval is set between each pair of adjacent gradations. In the present embodiment, the angular interval is set to one degree, and the seven gradations 62a to 62g correspond to the respective valgus angles θ from three degrees to nine degrees. That is, the indicator 50 includes the plurality of gradations 62a to 62g, which correspond to the plurality of (in the present embodiment, seven) intended valgus angles θ, respectively. In addition, among the plurality of gradations 62a to 62g, their visual elements such as the thickness, shape, color, etc., are varied between each pair of adjacent gradations in order to prevent false visual recognition. In the present embodiment, the central gradation 62d, the outermost gradation 62a, and the innermost gradation 62g are each formed to have a thicker straight shape, and the two gradations 62c and 62e disposed at both sides of the central gradation 62d are each formed to have a thinner straight shape. The remaining gradations 62b and 62f are each formed to have a straight shape with a plurality of protrusions formed thereon such that the protrusions protrude to both sides in the gradation width direction (i.e., formed to have a shape similar to that of a dotted line).

It should be noted that, alternatively, the gradations 62a to 62g may be formed as protrusions on or grooves in the surface of the first plate-shaped portion 56, or may be marked on the surface of the first plate-shaped portion 56 by use of a coating material. Moreover, each of the gradations 62a to 62g may be formed to have a shape different from a linear shape. For example, each of the gradations 62a to 62g may have a dotted shape or may be a geometric design. The plurality of gradations 62a to 62g shown in Fig. 7, which are provided in the second plate-shaped portion 58, are formed in the same manner as the plurality of gradations 62a to 62g provided in the first plate-shaped portion 56.

As shown in Fig. 7, the first plate-shaped portion 56 and the second plate-shaped portion 58 are joined to the spacer 60 by use of the female screw members 64a and the male screw members 64b. A protrusion 66 protruding toward the supporting pillar 26 (Fig. 4) is formed on the outer side surface of the spacer 60. A hole 66a is formed in the protrusion 66. One end of the coupling portion 54 is inserted in and fixed to the hole 66a.

As shown in Fig. 7, the first plate-shaped portion 56 and the second plate-shaped portion 58 (not shown) of the indicator 50 are disposed parallel to the frontal plane such that, when viewed in a direction perpendicular to the frontal plane, the first plate-shaped portion 56 and the second plate-shaped portion 58 obscure the femoral head I. Accordingly, if an image of the femoral head I and the indicator 50 is captured by use of an X-ray image intensifier described below in the direction perpendicular to the frontal plane (i.e., the vertical direction), the gradations 62a to 62g of the first plate-shaped portion 56 and the gradations 62a to 62g of the second plate-shaped portion 58 coincide with each other in the captured image. If these gradations do not coincide with each other in the captured image, it can be determined that the facing direction of the camera deviates from the direction perpendicular to the frontal plane (i.e., deviates from a proper direction). On the other hand, in a case where the facing direction of the camera of the X-ray image intensifier described below can be precisely fixed to the direction perpendicular to the frontal plane, if the gradations 62a to 62g of the first plate-shaped portion 56 and the gradations 62a to 62g of the second plate-shaped portion 58 do not coincide with each other in the captured image, it can be determined that the indicator 50 is not parallel to the frontal plane.

Specifically, in the present embodiment, a "first reference portion" and a "second reference portion" for determining whether or not the image-capturing direction is the proper direction and for determining whether or not the orientation of the indicator 50 is the proper orientation are formed by the gradations 62a to 62g of the first plate-shaped portion 56 and the gradations 62a to 62g of the second plate-shaped portion 58, which are used for measuring the valgus angle θ. It should be noted that the "first reference portion" and the "second reference portion" may be formed independently of the gradations 62a to 62g.

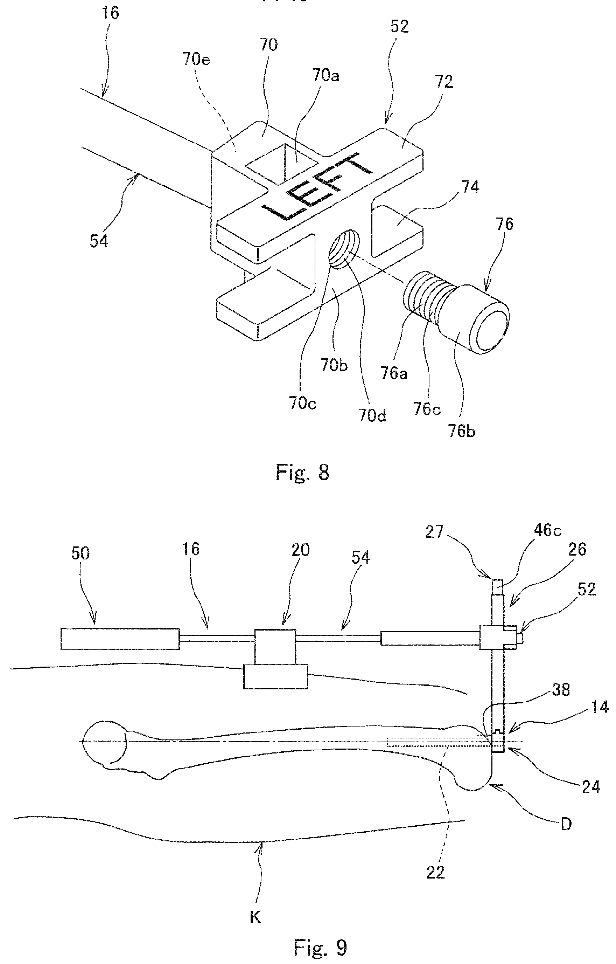

As shown in Fig. 8, the connection portion 52 (the first connection portion) includes: a connection portion body 70, which is substantially a rectangular parallelepiped; a plate-shaped first display portion 72 formed at the upper part of the connection portion body 70; a plate-shaped second display portion 74 formed at the lower part of the connection portion body 70; and a fixing member 76. A substantially quadrangular through-hole 70a, in which the supporting pillar 26 (Fig. 3) of the fixing part 14 is inserted, is formed in the connection portion body 70 in a manner extending vertically. A through-hole 70c, which extends from an outer side surface 70b of the connection portion body 70 to reach the inner surface of the through-hole 70a, is formed in the connection portion body 70. A female screw 70d is formed on the inner peripheral surface of the through-hole 70c. An outer side surface 70e of the connection portion body 70 at the indicator 50 (Fig. 4) side is formed to be perpendicular to the central gradation 62d (Fig. 4). A connection portion such as a hole or a protrusion to which the other end of the coupling portion 54 is connected is formed in or on the outer side surface 70e although it is not shown in the drawings. The supporting pillar body 40 (Fig. 5) and the through-hole 70a (Fig. 8) both have a non-round cross section. This makes it possible to prevent the connection portion 52 from rotating about the supporting pillar 26, and thereby the angles formed between the medullary rod 22 and the gradations 62a to 62g when seen in plan view can be kept to fixed angles.

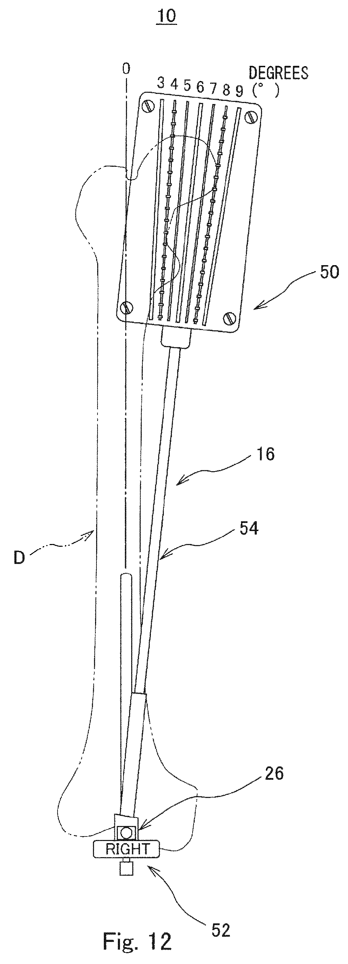

As shown in Fig. 8, letters "LEFT" are marked on the upper surface of the first display portion 72, and letters "RIGHT" (Fig. 12) are marked on the lower surface of the second display portion 74. As shown in Fig. 4, when the letters "LEFT" are seen on the upper surface of the connection portion 52, the connection portion 52 is in the state of being applicable to the left-side femur D. On the other hand, as shown in Fig. 12, when the letters "RIGHT" are seen on the upper surface of the connection portion 52, the connection portion 52 is in the state of being applicable to the right-side femur D.

As shown in Fig. 8, the fixing member 76 includes a shaft 76a and a head 76b. The head 76b is formed at one end of the shaft 76a in its axial direction. A male screw 76c is formed on the outer peripheral surface of the shaft 76a. The shaft 76a is formed to have a length that is greater than the length of the through-hole 70c formed in the connection portion body 70. When the male screw 76c is screwed into the female screw 70d of the connection portion body 70, the distal end of the shaft 76a protrudes into the through-hole 70a and comes into contact with the outer side surface of the supporting pillar 26 (Fig. 3) inserted in the through-hole 70a. In this manner, the connection portion 52 is fixed to the supporting pillar 26 (Fig. 3).

As shown in Fig. 3, the guide support 18 includes: a connection portion 80 serving as a "second connection portion" connected to the fixing part 14; the support body 82 connected to the connection portion 80; and a position adjusting mechanism 86, which adjusts the mounting position of the bone resection guide 11 (Fig. 11) mounted to the support body 82. As shown in Fig. 5, the connection portion 80 includes a columnar first portion 88. On one end of the first portion 88 in its axial direction, a columnar second portion 90 and a columnar third portion 92 are continuously formed in the axial direction. A male screw 92a is formed on the outer peripheral surface of the third portion 92. A head 94 is formed on the other end of the first portion 88 in the axial direction. The head 94 receives rotational force imparted from, for example, hand fingers of a surgeon. At the time of connecting the connection portion 80 to the fixing part 14, the male screw 92a is screwed into the female screw 30b as a result of the rotational force being imparted from the head 94 to the connection portion 80.

The support body 82 shown in Fig. 3 includes: a connection mechanism (not shown) detachably connected to the connection portion 80; and a guide attaching portion 82a. The guide attaching portion 82a is configured such that the bone resection guide 11 (Fig. 11) is detachably attached thereto at a fixed position. The guide attaching portion 82a includes a recess 82b, in which the supporting pillar 26 is temporarily disposed. The position adjusting mechanism 86 is configured to adjust the position of the guide attaching portion 82a and the bone resection guide 11 (Fig. 11) attached thereto in relation to the support body 82 in accordance with the valgus angle θ.

In the present embodiment, the gradations 62a to 62g of the measuring part 16 (Fig. 4) are provided corresponding to the respective valgus angles θ from three to nine degrees. Accordingly, the position adjusting mechanism 86 is configured to make adjustments over seven stages, i.e., capable of adjusting the aforementioned position to each of the valgus angles θ corresponding to the respective gradations 62a to 62g. In a case where the valgus angle θ of the femur D is six degrees, the adjustment stage of the position adjusting mechanism 86 at which the bone resection guide 11 (Fig. 11) can be properly positioned relative to the femur D is the fourth stage. Accordingly, in a case where the valgus angle θ of the femur D is five degrees, if the adjustment stage of the position adjusting mechanism 86 is set to the fourth stage, the position of the bone resection guide 11 (Fig. 11) deviates from the proper position by one degree. Therefore, in this case, the adjustment stage of the position adjusting mechanism 86 is set to the third stage, which is shifted from the fourth stage by one degree. It should be noted that the number of adjustment stages of the position adjusting mechanism 86 need not be the same as the number of gradations 62a to 62g (in the present embodiment, seven stages). For example, the number of adjustment stages may be set to 13 such that the stages correspond not only to the gradations but also to middle positions that are each located between two adjoining gradations.

Fig. 9 is a side view showing a state where the fixing part 14 and the measuring part 16 are positioned relative to the femur D. As shown in Fig. 9, the supporting member 20 supports the indicator 50 and the coupling portion 54 of the measuring part 16 on a femoral region K of the patient A. As shown in Fig. 3, the supporting member 20 includes: a plate-shaped curved portion 98 curved along the femoral region K (Fig. 9); and a support base 100 formed to protrude upward from the upper surface of the curved portion 98. The support base 100 includes a pair of sandwiching pieces 100a and 100b formed of a flexible material such as synthetic resin. A substantially U-shaped accommodating portion 102, which accommodates and holds the coupling portion 54 in a sandwiching manner, is formed at the upper part of the pair of sandwiching pieces 100a and 100b. When accommodating the coupling portion 54 in the accommodating portion 102, the sandwiching pieces 100a and 100b are elastically deformed in a direction in which the sandwiching pieces 100a and 100b are spaced apart from each other.

### (Method of Positioning Bone Resection Guide)

At the time of resecting the femur distal end Db in the artificial knee joint replacement surgery, first, a hole (not shown) is formed by a drill in the intercondylar region Q (Fig. 1) of the femur distal end Db in a direction parallel to the femoral anatomical axis L2. Subsequently, as shown in Fig. 9, the medullary rod 22 is inserted into the femur D through the hole, such that the medullary rod 22 is parallel to the femoral anatomical axis L2 (Fig. 1) and the supporting pillar 26 is perpendicular to the frontal plane, and thereby the pin 38 is stuck into the femur D. In this step, whether the supporting pillar 26 is perpendicular to the frontal plane may be confirmed by use of a level indicator.

As shown in Fig. 9, after the fixing part 14 is fixed to the femur D, the measuring part 16 is connected to the fixing part 14. Specifically, as shown in Fig. 4, in the case of using the bone resection guide positioning apparatus 10 on the left-side femur D, the measuring part 16 is connected to the supporting pillar 26 such that the letters "LEFT" appear on the upper side. On the other hand, as shown in Fig. 12, in the case of using the bone resection guide positioning apparatus 10 on the right-side femur D, the measuring part 16 is connected to the supporting pillar 26 such that the letters "RIGHT" appear on the upper side. By adjusting the position of the connection portion 52 relative to the supporting pillar 26 in the vertical direction and the position where the coupling portion 54 is supported by the supporting member 20, both the upper and lower surfaces of the indicator 50 are disposed parallel to the frontal plane. At the time, whether both the upper and lower surfaces of the indicator 50 are parallel to the frontal plane may be confirmed by use of a level indicator.

When the adjustment of the orientation of the indicator 50 is completed, an image of the indicator 50 and the femoral head I positioned therebelow is captured in a direction perpendicular to the frontal plane by use of an X-ray image intensifier that is not shown. Then, the image as shown in Fig. 4, in which the indicator 50 is superimposed on the femoral head I, is displayed on a monitor. Based on the positional relationship between the femoral head center P1 and the indicator 50, the valgus angle θ is measured. In the example of measurement shown in Fig. 4, the gradation 62c coincides with the femoral head center P1. Accordingly, the result of the measurement of the valgus angle θ is five degrees. When looking at the image on the monitor, if the gradations 62a to 62g of the first plate-shaped portion 56 (i.e., the first reference portion) and the gradations 62a to 62g of the second plate-shaped portion 58 (i.e., the second reference portion) do not coincide with each other in the image, the rotation position is adjusted such that the gradations 62a to 62g of the first plate-shaped portion 56 and the gradations 62a to 62g of the second plate-shaped portion 58 coincide with each other, or the position of the camera is adjusted.

Fig. 10 is a side view showing a state where the bone resection guide 11 is mounted to the guide support 18. When the measurement of the valgus angle θ is completed, the measuring part 16 shown in Fig. 9 is removed from the fixing part 14, and also, the supporting member 20 is removed. Then, as shown in Fig. 10, the guide support 18 is connected to the base 24. Specifically, first, the connection portion 80 is connected to the base 24. Thereafter, the support body 82 is connected to the connection portion 80. At the time, the supporting pillar 26 of the fixing part 14 is still kept connected to the base 24 although it. is not shown in the drawing. Since the supporting pillar 26 is disposed in the recess 82b of the guide attaching portion 82a, the supporting pillar 26 will not be a hindrance.

In order to improve the precision of positioning of the bone resection guide 11 relative to the femur D, it is desirable that the axis of the connection portion 80 be perpendicular to the axis of the supporting pillar 26. Therefore, in the present embodiment, a perpendicularity indicator (not shown) is additionally installed on the supporting pillar 26, and by use of the perpendicularity indicator, whether the axis of the connection portion 80 is perpendicular to the axis of the supporting pillar 26 is confirmed. At the time of connecting the support body 82 to the connection portion 80, the supporting pillar 26 serves as a reference for positioning the support body 82.

As shown in Fig. 10, when the guide support 18 is fully connected to the base 24, the supporting pillar 26 (Fig. 5) of the fixing part 14 is detached from the base 24. Thereafter, the bone resection guide 11 is attached to the guide attaching portion 82a of the support body 82. Subsequently, the position adjusting mechanism 86 is operated to adjust the mounting position of the bone resection guide 11 mounted to the support body 82 in accordance with the valgus angle θ, and thereby the bone resection guide 11 is positioned at a proper position on the femur D. In a case where the femoral head center P1 shown in Fig. 4 is disposed at a middle position between two adjoining gradations (e.g., a position indicating 5.5 degrees) and none of the adjustment stages of the position adjusting mechanism 86 corresponds to the middle position, the positioning of the bone resection guide 11 (Fig. 11) is difficult. In this case, another bone resection guide (not shown) including a guide portion that is, when seen in plan view, inclined relative to the guide portion 11b of the bone resection guide 11 (Fig. 11) by 0.5 degrees may be used. By use of such a bone resection guide, the bone resection guide can be precisely positioned at the proper position on the femur D without changing the configuration of the guide support 18.

As shown in Fig. 10, when the positioning of the bone resection guide 11 relative to the femur D is completed, the bone resection guide 11 is fixed to the femur D by use of fixing pins 104. Specifically, the fixing pins 104 are inserted in a plurality of respective through-holes 11a formed in the bone resection guide 11 (Fig. 10 shows only one of the fixing pins 104), and thereby the fixing pins 104 are stuck into the femur D. When the fixing of the bone resection guide 11 to the femur D is completed, as shown in Fig. 11, after the fixing part 14 and the guide support 18 are fully removed from the femur D, the femur distal end Db is cut out by the bone saw 13 in such a manner that the blade 13a of the bone saw 13 is moved along the guide portion 11b of the bone resection guide 11.

According to the present embodiment, the above-described configuration provides advantageous effects as follows. Specifically, as shown in Fig. 4, the valgus angle θ can be readily and precisely measured by use of the measuring part 16, and based on the valgus angle θ, the bone resection guide 11 can be precisely positioned at the proper position on the femur D. Therefore, expensive surgery assisting equipment, such as a navigation system, is unnecessary. This makes it possible to reduce the cost of the artificial knee joint replacement. In addition, an image for measuring the valgus angle θ can be obtained within a short period of time by use of an X-ray image intensifier. This makes it possible to reduce the exposure dose. As shown in Fig. 10, at the time of mounting the bone resection guide 11 to the support body 82, the supporting pillar 26 can be removed from the base 24. Therefore, the supporting pillar 26 will not be a hindrance.

As shown in Fig. 10, by operating the position adjusting mechanism 86 in accordance with the valgus angle θ, the mounting position of the bone resection guide 11 mounted to the support body 82 can be readily and precisely adjusted in accordance with the valgus angle θ, and the bone resection guide 11 can be precisely positioned at the proper position on the femur D.

As shown in Fig. 4, the indicator 50 includes the plurality of gradations 62a to 62g, which correspond to the plurality of intended valgus angles θ, respectively. This makes it possible to readily measure the valgus angle θ. Since the indicator 50 and the connection portion 52 (the first connection portion) are coupled together by the rod-shaped coupling portion 54, the entire measuring part 16 can be formed to be small-sized and light-weighted. Therefore, the measuring part 16 can be made easy to handle. As shown in Fig. 7, the plurality of gradations 62a to 62g are embedded in each of the first plate-shaped portion 56 and the second plate-shaped portion 58. This makes it possible to prevent damage to the gradations 62a to 62g.

As shown in Fig. 4 and Fig. 12, by inverting the upper and lower sides of the measuring part 16, the measuring part 16 can be used for both the right-side femur D and the left-side femur D. Therefore, the manufacturing cost can be reduced compared to a case where two measuring parts are used.

As shown in Fig. 7, whether or not the image-capturing direction is the proper direction and whether or not the orientation of the indicator 50 is the proper orientation can be readily determined by merely checking whether or not the gradations 62a to 62g of the first plate-shaped portion 56 (i.e., the first reference portion) and the gradations 62a to 62g of the second plate-shaped portion 58 (i.e., the second reference portion) coincide with each other. In addition, the manufacturing cost can be reduced compared to a case where the first reference portion and the second reference portion are formed independently of the gradations 62a to 62g.

As shown in Fig. 9, by the locking of the pin 38 to the femur D, the fixing part 14 can be prevented from rotating about the axis of the medullary rod 22, and thereby shifts in the positions of the measuring part 16 and the guide support 18 can be prevented, which makes it possible to precisely measure the valgus angle 0.

As shown in Fig. 5, rotational force for detaching the male screw 46b (the first screw) of the connecting member 27 from the female screw 32c (the second screw) of the base 24 can be imparted to the connecting member 27 from the head 46c provided at the upper end of the connecting member 27. Accordingly, even during the surgery, the supporting pillar 26 can be readily detached from the base 24.

### (Variations)

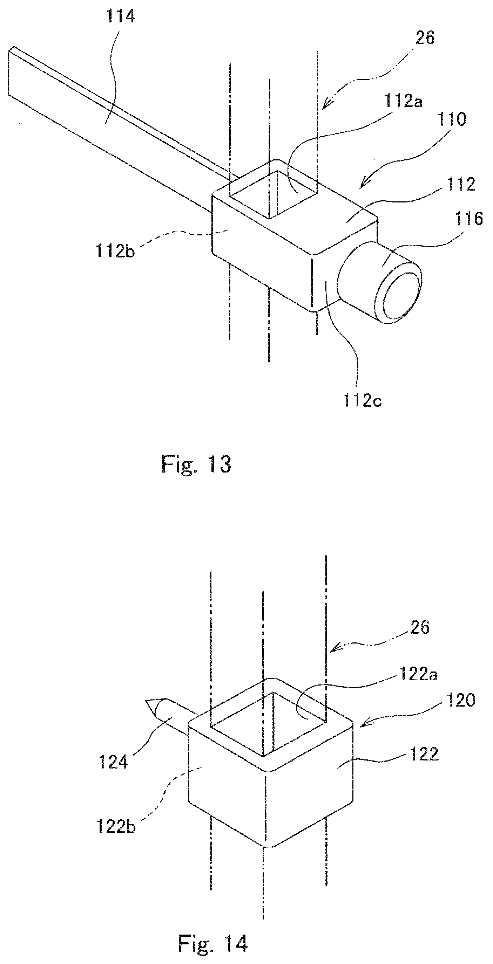

As shown in Fig. 5, in the above-described embodiment, the pin 38 provided on the base 24 is used as the "locking portion" for preventing the fixing part 14 from rotating about the axis of the medullary rod 22. However, as an alternative, a locking portion 110 shown in Fig. 13 or a locking portion 120 shown in Fig. 14 may be used in place of the pin 38.

The locking portion 110 shown in Fig. 13 includes: an attachment 112 including a through-hole 112a in which the supporting pillar 26 is inserted; a rod-shaped or protruding contacting portion 114 formed in a manner extending from an outer side surface 112b of the attachment 112 in the same direction as the extending direction of the medullary rod 22 (Fig. 3), the contacting portion 114 coming into contact with the outer surface of the femur D (Fig. 1) from above; and a fixing member 116 fixing the attachment 112 to the supporting pillar 26. A through-hole extending from an outer side surface 112c of the attachment 112 to reach the inner surface of the through-hole 112a is formed in the attachment 112 although the through-hole is not shown in the drawings. A female screw into which a male screw of the fixing member 116 is screwed is formed on the inner peripheral surface of the through-hole. When the contacting portion 114 is positioned on the Whiteside line of the femur distal end Db, the supporting pillar 26 is perpendicular to the frontal plane. Therefore, the perpendicularity of the supporting pillar 26 relative to the frontal plane can be confirmed based on the positional relationship between the Whiteside line and the contacting portion 114.

The locking portion 120 shown in Fig. 14 includes: an attachment 122 including a through-hole 122a in which the supporting pillar 26 is inserted; and a pin 124 formed in a manner extending from an outer side surface 122b of the attachment 122 in the same direction as the extending direction of the medullary rod 22 (Fig. 3), the pin 124 being stuck into the femur D (Fig. 1). The attachment 122 is movable in the axial direction of the supporting pillar 26. However, since the pin 124 is stuck into the femur D (Fig. 1), the attachment 122 does not easily move in the axial direction relative to the supporting pillar 26. Therefore, the locking portion 120 does not require a fixing member for fixing the attachment 122 to the supporting pillar 26. For this reason, the locking portion 120 can be readily manufactured.

### Reference Signs List

- 10: bone resection guide positioning apparatus
- 14: fixing part
- 16: measuring part
- 18: guide support
- 22: medullary rod
- 24: base
- 26: supporting pillar
- 50: indicator
- 52: first connection portion
- 82: support body
- 80: second connection portion

## Claims

1. A bone resection guide positioning apparatus for positioning a bone resection guide on a femur, the bone resection guide guiding a bone saw, the bone resection guide positioning apparatus comprising:
a fixing part including a medullary rod configured to be inserted into the femur, a base provided at one end of the medullary rod and configured to be disposed in proximity to a distal end of the femur, and a supporting pillar detachably connected to the base;
a measuring part including a first connection portion connected to the supporting pillar and an indicator coupled to the first connection portion, the indicator being configured to indicate a valgus angle formed by a femoral functional axis and a femoral anatomical axis based on a positional relationship between the indicator and a femoral head center, the femoral functional axis connecting the femoral head center and a knee joint center; and
a guide support including a second connection portion connected to the base and a support body connected to the second connection portion, to which support body the bone resection guide is detachably mounted, such that a mounting position of the bone resection guide mounted to the support body is adjustable.

2. The bone resection guide positioning apparatus according to claim 1, wherein
the indicator includes a plurality of gradations corresponding to a plurality of intended valgus angles, respectively.

3. The bone resection guide positioning apparatus according to claim 2, wherein
the indicator includes a plate-shaped portion disposed such that, when seen in a direction perpendicular to a frontal plane, the plate-shaped portion obscures the femoral head, and
the plurality of gradations are embedded in the plate-shaped portion.

4. The bone resection guide positioning apparatus according to any one of claims 1 to 3, wherein
the measuring part is configured to be usable for both right-side,and left-side femurs by inverting upper and lower sides of the measuring part.

5. The bone resection guide positioning apparatus according to any one of claims 1 to 4, wherein
the indicator includes a first reference portion and a second reference portion spaced apart from each other in a direction perpendicular to a frontal plane, and
the first reference portion and the second reference portion coincide with each other when the indicator is viewed in the direction perpendicular to the frontal plane.

6. The bone resection guide positioning apparatus according to claim 5, wherein
the first reference portion and the second reference portion are each formed by gradations that are used for valgus angle measurement.

7. The bone resection guide positioning apparatus according to any one of claims 1 to 6, wherein
the fixing part is provided with a locking portion configured to be locked to the femur.

8. The bone resection guide positioning apparatus according to any one of claims 1 to 7, wherein
the supporting pillar includes a through-hole formed in a manner extending in an axial direction of the supporting pillar, and
the supporting pillar and the base are connected by use of a connecting member inserted in the through-hole.

9. The bone resection guide positioning apparatus according to any one of claims 1 to 8, wherein
the guide support includes a position adjusting mechanism that adjusts the mounting position of the bone resection guide mounted to the support body.
